# DEMANDE DE BREVET EUROPEEN

(11) **EP 0 611 172 A1**
(43) Date de publication de la demande: **17.08.1994**
(21) Numéro de dépôt: 94400042.1
(22) Date de dépôt: 06.01.1994
(51) Int. Cl.: A61M 1/16

(54) **Dispositif de préparation d'une solution par dissolution d'au moins une substance dans un liquide**

(30) Priorité: 01.02.1993 FR 9301255
(71) Demandeur: Lascombes, Jean-Jacques, F-31000 Toulouse (FR); Courtiade, Philippe, F-31280 Aigrefeuille (FR)
(72) Inventeur: Lascombes, Jean-Jacques, F-31000 Toulouse (FR); Courtiade, Philippe, F-31280 Aigrefeuille (FR)
(74) Mandataire: Morelle, Guy Georges Alain

(57) **Abrégé**

La présente invention concerne les dispositifs de préparation d'une solution par dissolution d'au moins une substance dans un liquide.

Le dispositif selon l'invention se caractérise essentiellement par le fait qu'il comporte un circuit 1 de liquide 2 comprenant une conduite 3, une cartouche 4 de la substance 5 pour délivrer un soluté et une conduite 9, un circuit 11 du liquide 2, un circuit 14 relié aux deux circuits 1 et 11, des moyens 20 pour commander la circulation du liquide dans l'un des circuits 1 et 11, des moyens 30 pour mesurer la composition du soluté dans la conduite 9 et délivrer un premier signal, des moyens 40 pour mesurer le débit de fluide dans le circuit 1 et délivrer un deuxième signal, des moyens 50 pour mesurer le débit de fluide dans le circuit 11 et délivrer un troisième signal, et un organe de traitement 60 qui, en fonction de ces trois signaux, commande les moyens 20.

Aplication : notamment pour des traitements tels que l'hémodialyse, l'hémodiafiltration et l'hémofiltration.

## Description

La présente invention concerne les dispositifs de préparation d'une solution par dissolution d'au moins une substance dans un liquide, qui trouvent une application particulièrement avantageuse dans le domaine médical, pour la préparation de produits pour dialyse, notamment à base de bicarbonate, destinés à être utilisés pour des traitements tels que l'hémodialyse, l'hémodiafiltration et l'hémofiltration.

Des dispositifs de préparation d'une solution par dissolution d'au moins une substance dans un liquide sont notamment connus par le EP-A-0 278 100.

Ces dispositifs connus comportent essentiellement une cartouche contenant une substance soluble dans l'eau, une première conduite de fluide reliée à une source d'eau et apte à délivrer à sa sortie une solution aqueuse de la substance, une deuxième conduite de fluide reliée à une source d'eau et à une entrée de la cartouche afin de produire, en sortie de celle-ci, un soluté, une troisième conduite de fluide communiquant avec une sortie de la cartouche et avec un point de mélange situé dans la première conduite de fluide pour conduire le soluté de la cartouche à la première conduite de fluide afin de le mélanger avec l'eau de la première conduite de fluide, de manière à produire, à la sortie de la première conduite de fluide, la solution aqueuse cherchée, des moyens de mesure placés dans la première conduite de fluide en aval du point de mélange, pour mesurer la composition de la solution obtenue à la sortie de la première conduite de fluide, et un régulateur placé dans la troisième conduite de fluide et répondant aux moyens de mesure, pour régler le débit du soluté en sortie de la cartouche.

Ces dispositifs donnent de bons résultats. Cependant, dans de nombreux domaines d'application, notamment dans le domaine médical, il est nécessaire, d'une part que la composition de la solution obtenue en sortie soit la plus constante possible, ainsi que son débit, et d'autre part que le régulateur réponde le plus rapidement possible selon les valeurs des mesures effectuées en aval du point de mélange.

Or, il est constaté que, lorsque la prise de mesures s'effectue après le point de mélange comme dans les dispositifs de l'art antérieur, notamment dans le dispositif décrit dans le EP-A-0 278 100, la constante de temps pour la régulation est trop grande et la composition de la solution en sortie du dispositif subit des variations qui sont préjudiciables à certains traitements.

La présente invention a ainsi pour but de pallier les inconvénients mentionnés ci-dessus et de réaliser un dispositif de préparation d'une solution par dissolution d'au moins une substance dans un liquide, qui trouve une application particulièrement avantageuse pour la préparation de produits pour dialyse, notamment à base de bicarbonate, destinés à être utilisés dans le domaine médical pour des traitements tels que l'hémodialyse, l'hémodiafiltration et l'hémofiltration, qui soit d'une structure relativement simple, qui donne en sortie une solution ayant, notamment, une concentration constante avec une grande précision et un débit bien contrôlé, et qui permette de régler très rapidement, si nécessaire, la valeur de ce débit.

Plus précisément, la présente invention a pour objet un dispositif de préparation d'une solution par dissolution d'au moins une substance dans un liquide, comportant :
un premier circuit fluidique apte à véhiculer ledit liquide, ledit premier circuit fluidique comprenant, montées en série, une première conduite, une cartouche contenant ladite substance soluble dans ledit liquide, l'entrée de ladite cartouche étant reliée à la sortie de ladite première conduite pour délivrer à sa sortie un soluté de ladite substance dans ledit liquide, et une seconde conduite dont l'entrée est reliée à la sortie de ladite cartouche,
un deuxième circuit fluidique apte à véhiculer ledit liquide,
un troisième circuit fluidique dont l'entrée est reliée aux sorties de ladite seconde conduite et dudit deuxième circuit fluidique, et
des moyens pour mesurer la composition dudit soluté véhiculé dans ladite seconde conduite et délivrer un premier signal représentatif de la valeur de cette mesure,
caractérisé par le fait qu'il comporte en outre :
des moyens pour commander la circulation dudit liquide dans ledit deuxième circuit fluidique en fonction d'un signal de commande,
des moyens pour mesurer le débit de fluide dans ledit premier circuit fluidique et délivrer un deuxième signal représentatif de la valeur de ce débit,
des moyens pour mesurer le débit de fluide dans ledit deuxième circuit fluidique et délivrer un troisième signal représentatif de la valeur de ce débit, et
un organe de traitement recevant lesdits premier, deuxième et troisième signaux et apte à délivrer en sortie un quatrième signal élaboré en fonction de la valeur de cesdits premier, deuxième et troisième signaux, cedit quatrième signal constituant ledit signal de commande appliqué auxdits moyens pour commander la circulation dudit liquide dans ledit deuxième circuit fluidique.

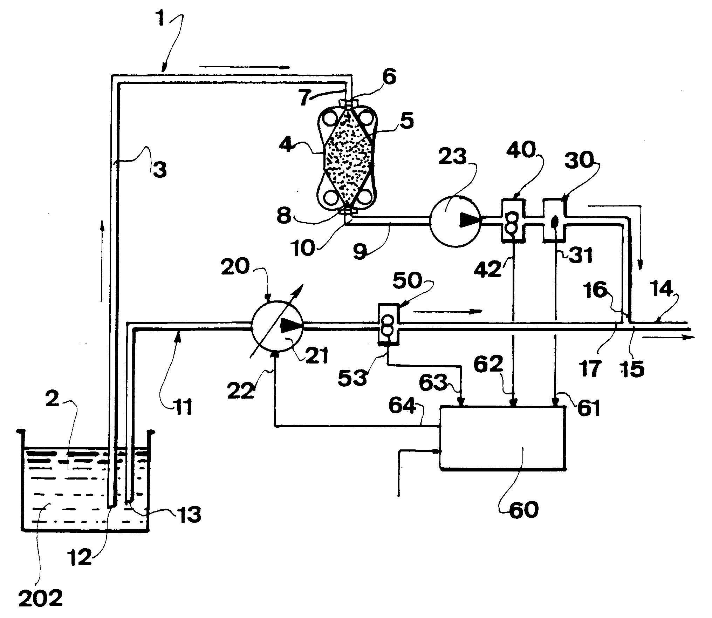

D'autres caractéristiques et avantages de la présente invention apparaîtront au cours de la description suivante donnée en regard du dessin annexé à titre illustratif, mais nullement limitatif, dans lequel la figure unique représente, sous forme schématique, un mode de réalisation préférentiel d'un dispositif selon l'invention, de préparation d'une solution par dissolution d'au moins une substance dans un liquide.

La figure unique représente un dispositif de préparation d'une solution par dissolution d'au moins une substance dans un liquide, par exemple du bicarbonate dans de l'eau.

Ce dispositif comporte un premier circuit fluidique 1 apte à véhiculer le liquide 2, ce premier circuit 1 comprenant, montées en série, une première conduite 3, une cartouche 4 contenant la substance 5 soluble dans le liquide, dont l'entrée 6 est reliée à la sortie 7 de la première conduite 3 et qui est apte à délivrer à sa sortie 8 un soluté de la substance 5 dans le liquide 2, et une seconde conduite 9 dont l'entrée 10 est reliée à la sortie 8 de la cartouche 4.

Le dispositif comporte aussi un deuxième circuit fluidique 11 apte à véhiculer, dans le mode de réalisation illustré, le même liquide 2. Mais il est concevable que les liquides véhiculés dans les deux circuits fluidiques 1 et 11 pourraient être de composition différente.

Quand le liquide est le même pour les deux circuits fluidiques 1 et 11, il est avantageux que les deux entrées 12 de la première conduite 3 et 13 du deuxième circuit fluidique 11 soient toutes les deux reliées à une même source 202 de ce liquide.

Le dispositif comporte ensuite un troisième circuit fluidique 14 dont l'entrée 15 est reliée à la sortie 16 de la seconde conduite 9 et à la sortie 17 du deuxième circuit fluidique 11, de façon que les fluides qui arrivent respectivement à la sortie 16 de la deuxième conduite 9 et à la sortie 17 du deuxième circuit fluidique 11 se mélangent et que leur mélange constitue la solution cherchée destinée à être véhiculée dans ce troisième circuit fluidique.

Selon une caractéristique de l'invention, le dispositif comporte en outre des moyens 20 pour commander la circulation du liquide dans au moins l'un des deux premier et deuxième circuits fluidiques en fonction d'un signal de commande, des moyens 30 pour mesurer la composition du soluté véhiculé dans la seconde conduite et aptes à délivrer à leur sortie 31 un premier signal représentatif de la valeur de cette mesure, des moyens 40 pour mesurer le débit de fluide dans le premier circuit fluidique 1 et aptes à délivrer à leur sortie 42 un deuxième signal représentatif de la valeur de ce débit, des moyens 50 pour mesurer le débit de fluide dans le deuxième circuit fluidique 11 et aptes à délivrer à leur sortie 53 un troisième signal représentatif de la valeur de ce débit, et un organe de traitement 60 recevant respectivement sur ses entrées 61, 62, et 63 les premier, deuxième et troisième signaux et apte à délivrer en sortie 64 un quatrième signal élaboré en fonction de la valeur de ces premier, deuxième et troisième signaux, ce quatrième signal constituant le signal de commande qui est appliqué aux moyens 20 pour commander la circulation du liquide dans l'un des deux premier 1 et deuxième 11 circuits fluidiques.

Selon une réalisation avantageuse du dispositif, les moyens 20 pour commander la circulation du liquide dans au moins l'un des deux premier et deuxième circuits fluidiques en fonction du signal de commande délivré par l'organe de traitement 60 sont avantageusement situés, comme dans la réalisation illustrée, dans le deuxième circuit fluidique 11, c'est-à-dire le circuit fluidique qui ne comprend pas la cartouche 4. Cette réalisation est avantageuse car la perte de charge dans ce deuxième circuit 11 ne varie pas dans le temps comme cela se produit dans le premier circuit 1, et la régulation du débit dans ce deuxième circuit 11 est alors plus facile et surtout plus précise. En effet, la perte de charge dans le premier circuit 1 varie dans le temps au cours de la dissolution de la substance 5 contenue dans la cartouche 4 et il peut en outre se produire des phénomènes de cristallisation partielle qui limitent progressivement l'écoulement du fluide dans la cartouche, ce qui est équivalent à une augmentation de la perte de charge dans les premiers temps et à une diminution ensuite.

Ces moyens 20 sont préférentiellement constitués par une pompe à débit variable 21 commandable à partir, par exemple, d'une entrée de commande 22 sur laquelle peut être appliqué un signal de commande approprié, notamment celui qui est délivré à la sortie 64 de l'organe de traitement 60.

En outre, pour diminuer l'influence de la variation de la perte de charge engendrée par la dissolution de la substance 5 contenue dans la cartouche 4, le dispositif comporte avantageusement des moyens 23 pour réguler le débit dans le premier circuit fluidique 1, par exemple constitués par une pompe à débit constant qui est préférentiellement montée en série dans la deuxième conduite 9 du premier circuit 1.

Dans un mode de réalisation préférentiel, les moyens 40 pour mesurer le débit de fluide dans le premier circuit fluidique 1 et aptes à délivrer en sortie 42 un deuxième signal représentatif de la valeur de ce débit sont situés entre la pompe à débit constant 23 et l'entrée 15 du troisième circuit fluidique 14. De même, les moyens 50 pour mesurer le débit de fluide dans le deuxième circuit fluidique 11 et aptes à délivrer en sortie 53 un troisième signal représentatif de la valeur de ce débit sont situés entre la pompe à débit variable 21 et l'entrée 15 de ce troisième circuit fluidique 14.

Ces moyens 40 et 50 sont constitués par des débitmètres, par exemple magnétiques, mécaniques, ou fluidiques, etc, qui sont bien connus en eux-mêmes. Il en existe de nombreux types et ils seront choisis en fonction de la précision désirée et de la qualité des solutés et des solutions, en tenant compte des impératifs relatifs aux conditions d'utilisation du dispositif, par exemple du degré de stérilisation du dispositif et du soluté qui est souhaité.

Quant aux moyens 30 pour mesurer la composition du soluté en sortie de la cartouche 4, ils sont avantageusement situés en série entre la pompe à débit constant 23 et l'entrée 15 du circuit 14. Ces moyens 30 sont connus en eux-mêmes et peuvent par exemple être constitués par un capteur de mesure de la conductivité (ou de la résistivité, ce qui est équivalent), ou de tout autre paramètre qui caractérise la composition et/ou la concentration, selon les cas, du soluté.

Le dispositif décrit ci-dessus fonctionne de la façon suivante :

Le liquide 2, en l'occurrence de l'eau qui provient d'une même source 202, est envoyé dans les deux circuits fluidiques 1 et 11, la circulation de ce fluide étant produite par les deux pompes 21 et 23. L'eau amenée par la première conduite 3 du premier circuit fluidique 1 arrive dans la cartouche 4 et y dissout la substance 5 pour produire un soluté en sortie 8 de la cartouche. Ce soluté est entraîné dans la seconde conduite 9 du premier circuit fluidique 1 pour être mélangé, à l'entrée 15 du troisième circuit fluidique 14, avec l'eau qui arrive à la sortie 17 du deuxième circuit fluidique 11.

La combinaison de ces deux flux permet d'ajuster à une valeur désirée la concentration de la substance dissoute dans la solution véhiculée dans le troisième circuit fluidique 14.

Dans l'art antérieur, le contrôle de cette concentration s'effectue dans ce troisième circuit 14. En conséquence, le temps de réponse pour ajuster la concentration du soluté en fonction de la mesure de celle du flux total dans le troisième circuit 14 peut être relativement important parce qu'il dépend, notamment, de la longueur de la conduite 9 entre la sortie 8 de la cartouche et l'entrée 15 du troisième circuit fluidique 14.

Avec le dispositif selon l'invention, comme la mesure de la concentration est effectuée en 30 juste en sortie de la cartouche, une éventuelle variation de la valeur de la concentration du soluté est très rapidement détectée. Comme on connaît en outre, par les mesures effectuées avec les débitmètres 40 et 50, les valeurs des débits dans les premier et deuxième circuits, il est possible de régler presque instantanément la valeur de l'un des deux débits pour obtenir, dans le troisième circuit 14, une solution de concentration constante.

En fait, l'organe de traitement 60 délivre à sa sortie 64 un signal qui commande la pompe 21 à débit variable commandable pour régler le débit de fluide dans le deuxième circuit 11. En effet, la concentration de la solution dans le troisième circuit 14 est sensiblement proportionnelle à la concentration du soluté en sortie de la cartouche 4 et inversement proportionnelle à la somme des deux débits respectivement dans les circuits 1 et 11. En ajustant le débit dans le deuxième circuit 11, on obtient donc une concentration constante dans le circuit 14.

Par exemple, si la concentration du soluté diminue en sortie 8 de la cartouche, la pompe 21 va réduire son débit pour que la quantité de fluide arrivant à la sortie 17 du deuxième circuit 11 et qui se mélange avec le soluté "pauvre" donne dans le circuit 14 une solution finale à la concentration voulue. De même, si la concentration du soluté augmente en sortie 8 de la cartouche, cette augmentation aussitôt détectée permet à l'organe de traitement de commander la pompe 21 pour augmenter le débit de fluide dans le deuxième circuit 11 pour que la concentration de la solution finale dans le troisième circuit 14 soit constante.

Bien entendu, notamment quand un tel dispositif est utilisé dans le domaine médical, par exemple pour les applications définies au préambule de la présente description, il peut comporter en outre d'autres moyens, notamment pour filtrer les différents fluides et solutés, ainsi que des alarmes ou analogues pour la sécurité des patients.

## Revendications

1. Dispositif de préparation d'une solution par dissolution d'au moins une substance dans un liquide, comportant :
un premier circuit fluidique (1) apte à véhiculer ledit liquide (2), cedit premier circuit fluidique comprenant, montées en série, une première conduite (3), une cartouche (4) contenant ladite substance (5) soluble dans ledit liquide, l'entrée (6) de ladite cartouche étant reliée à la sortie (7) de ladite première conduite pour délivrer à sa sortie (8) un soluté de ladite substance dans ledit liquide, et une seconde conduite (9) dont l'entrée (10) est reliée à la sortie (8) de ladite cartouche,
un deuxième circuit fluidique (11) apte à véhiculer ledit liquide,
un troisième circuit fluidique (14) dont l'entrée (15) est reliée aux sorties (16, 17) de ladite seconde conduite et dudit deuxième circuit fluidique, et
des moyens (30) pour mesurer la composition dudit soluté véhiculé dans ladite seconde conduite (9) et délivrer un premier signal représentatif de la valeur de cette mesure,
***caractérisé par le fait qu***'il comporte en outre :
des moyens (20) pour commander la circulation dudit liquide dans ledit deuxième (11) circuit fluidique en fonction d'un signal de commande,
des moyens (40) pour mesurer le débit de fluide dans ledit premier circuit fluidique (1) et délivrer un deuxième signal représentatif de la valeur de ce débit,
des moyens (50) pour mesurer le débit de fluide dans ledit deuxième circuit fluidique (11) et délivrer un troisième signal représentatif de la valeur de ce débit, et
un organe de traitement (60) recevant lesdits premier, deuxième et troisième signaux et apte à délivrer en sortie (64) un quatrième signal élaboré en fonction de la valeur de cesdits premier, deuxième et troisième signaux, cedit quatrième signal constituant ledit signal de commande appliqué auxdits moyens (20) pour commander la circulation dudit liquide dans ledit deuxième circuit fluidique.

2. Dispositif selon la Revendication 1, ***caractérisé par le fait que*** les moyens (20) pour commander la circulation dudit liquide dans ledit deuxième circuit fluidique en fonction d'un signal de commande sont constitués par une pompe (21) à débit variable commandable montée en série dans ledit deuxième (11) circuit fluidique.

3. Dispositif selon l'une des Revendications 1 et 2, ***caractérisé par le fait qu***'il comporte en outre des moyens (23) pour réguler le débit de fluide dans ledit premier circuit fluidique (1).

4. Dispositif selon la Revendication 3, ***caractérisé par le fait que*** lesdits moyens (23) pour réguler le débit de fluide dans ledit premier circuit fluidique (1) sont montés en série sur ladite deuxième conduite (9).

5. Dispositif selon la Revendication 4, ***caractérisé par le fait que*** lesdits moyens pour réguler le débit de fluide dans ledit premier circuit fluidique montés en série sur ladite deuxième conduite sont constitués par une pompe à débit constant.

6. Dispositif selon les Revendications 1 et 5, ***caractérisée par le fait que*** lesdits moyens (30) pour mesurer la composition dudit soluté véhiculé dans ladite seconde conduite (9) et délivrer un premier signal représentatif de la valeur de cette mesure sont situés entre ladite pompe à débit constant (23) et l'entrée (15) dudit troisième circuit fluidique (14).

7. Dispositif selon l'une des Revendications 1 à 6, ***caractérisé par le fait que*** ledits moyens (40) pour mesurer le débit de fluide dans ledit premier circuit fluidique (1) et délivrer un deuxième signal représentatif de la valeur de ce débit sont situés entre ladite pompe à débit constant (23) et l'entrée (15) dudit troisième circuit fluidique (14).

8. Dispositif selon l'une des Revendications 1 à 7, ***caractérisé par le fait que*** lesdits moyens (50) pour mesurer le débit de fluide dans ledit deuxième circuit fluidique (11) et délivrer un troisième signal représentatif de la valeur de ce débit sont situés entre ladite pompe à débit variable (21) et l'entrée dudit troisième circuit fluidique.
